# FASCICULE DE BREVET EUROPEEN

(11) **EP 2 313 015 B1**
(45) Date de publication et mention de la délivrance du brevet: **26.08.2015**
(21) Numéro de dépôt: 09802510.9
(22) Date de dépôt: 28.07.2009
(51) Int. Cl.: A61B 17/34, A61B 17/00, A61B 19/00

(54) **DISPOSITIF DE POSITIONNEMENT D'UN OUTIL CHIRURGICAL DANS LE CORPS D'UN PATIENT**
VORRICHTUNG ZUR POSITIONIERUNG EINES CHIRURGISCHEN WERKZEUGS IM KÖRPER EINES PATIENTEN
DEVICE FOR POSITIONING A SURGICAL TOOL IN THE BODY OF A PATIENT

(30) Priorité: 29.07.2008 FR 0855189
(43) Date de publication de la demande: 27.04.2011
(73) Titulaire: Université Joseph Fourier - Grenoble 1, 38400 St. Martin d'Heres (FR); Universidad Autonoma Del Estado De Mexico, Toluca 50000 (MX)
(72) Inventeur: CINQUIN, Philippe, 38330 St Nazaire Les Eymes (FR); AVILA VILCHIS, Juan, Carlos, Toluca 50130 (MX); VILCHIS GONZALEZ, Adriana, Herlinda, Toluca 50130 (MX); CRUZ ABUD, Angel, Mariano, Toluca 50100 (MX); TROCCAZ, Jocelyne, 38320 Eybens (FR)
(74) Mandataire: Regimbeau
(86) Numéro de dépôt international: PCT/EP2009/059767
(87) Numéro de publication internationale: WO 2010/012744

(56) Documents cités:
- WO-A-2007/064739
- FR-A- 2 875 123
- FR-A1- 2 875 123
- US-A1- 2002 082 518
- US-A1- 2003 055 436
- US-A1- 2003 055 436

## Description

### DOMAINE DE L'INVENTION

La présente invention concerne un dispositif de positionnement d'un outil chirurgical dans le corps d'un patient.

### ARRIERE PLAN DE L'INVENTION

L'intérêt scientifique pour le développement de systèmes robotisés d'aide médicale a considérablement augmenté depuis les années 1990.

Plusieurs domaines médicaux ont vu l'apparition de systèmes génériques ou dédiés comme dans les cas du robot Da Vinci™ utilisé en laparoscopie, mais encore des systèmes pour la télé-opération ou pour une application cardiaque.

Les avantages de la chirurgie minimalement invasive ont contribué au développement de capteurs et d'actionneurs particuliers. Un panorama du développement de robots médicaux est fourni dans l'article «Medical Robotics in Computer-Integrated Surgery » de Taylor et al, IEEE Transactions on Robotics and Automation, Vol 19, No 5, Oct. 2003, où un nombre important de robots pour des spécialités médicales différentes est considéré.

En général, les sous-systèmes porte-outil (endoscope, laparoscope ou aiguille) ont un maximum de 4 degrés de liberté permettant l'orientation de l'outil, son insertion et sa rotation axiale.

L'orientation des outils à l'extérieur du corps du patient ainsi que leur insertion sont faites par des actionneurs.

Plusieurs sortes d'actionneurs ont été considérées pour les systèmes robotiques tels que les moteurs électriques, les moteurs ultrasonores, les moteurs piézo-électriques, les moteurs pneumatiques/hydrauliques harmoniques et planétaires ou les muscles artificiels de McKibben.

Les muscles artificiels utilisent l'air comprimé comme source d'énergie et sont utilisés en antagonisme ; cependant, leur taux de compression est faible et ils portent des parties métalliques non souhaitables dans le portique des appareils de tomographie ou de résonance magnétique.

Dans la chirurgie minimalement invasive ou dans des procédures de ponction, les outils chirurgicaux sont parfois orientés ou déplacés manuellement ou à l'aide d'actionneurs électriques qui en général sont incompatibles avec les environnements de tomographie et/ou de résonance magnétique, ou par d'autres actionneurs lents.

Plusieurs systèmes robotisés sont incompatibles avec les environnements mentionnés car ils ont été construits avec des matériaux métalliques, paramagnétiques ou diamagnétiques. Ces inconvénients sont liés à une conception mécanique ou à la façon dont les systèmes sont actionnés.

Un robot pneumatique léger et compatible avec les environnements de tomographie et de résonance magnétique est présenté dans l'article de E. Taillant et al, « CT and MR Compatible Light Puncture Robot : Architectural Design and First Experiments », Proceedings of Medical Image Computing and Computer Assisted Intervention (MICCAI), Lecture Notes in Computer Science, Springer Verlag, Vol. 3216, pp. 145-152, 2004. Ce système est adapté à la ponction thoracique et abdominale sous scanner ou résonance magnétique tout en étant interdépendant du corps du patient.

Ce robot permet d'orienter le porte-aiguille au moyen de deux rotations : une première rotation de la base du robot par rapport à une plateforme solidaire du corps du patient, à 360° autour d'un axe perpendiculaire au corps du patient, et une deuxième rotation du porte-aiguille, par rapport à la base, dans une plage angulaire limitée.

Ce dispositif d'orientation est constitué d'actionneurs pneumatiques classiques, c'est-à-dire comprenant chacun un piston coulissant dans un cylindre alimenté en air comprimé de part et d'autre du piston.

Chaque piston coopère avec une roue dentée, elle-même reliée à une vis sans fin, constituant ainsi un moteur pneumatique pas-à-pas.

De par sa conception, ce dispositif ne permet donc de positionner l'outil que selon une pluralité de positions discrètes.

Ce dispositif d'orientation est également relativement lent, avec une fréquence de l'ordre de 3 Hz.

Par ailleurs, un phénomène de glissement et d'adhérence (dit « stick-slip ») du piston dans la chambre a été observé lorsque le piston est immobile, conduisant à un mouvement abrupt lors de sa mise en mouvement et à la génération d'un retard dans la réponse de l'actionneur.

Enfin, un autre inconvénient de ce robot est son caractère « non compilant » : en raison de la rigidité de la chaîne cinématique, ce robot n'est pas en mesure d'absorber des efforts importants, de sorte que ceux-ci sont susceptibles de blesser le patient lorsque l'outil est à l'intérieur de son corps.

WO2007064739 divulgue un système selon le préambule de la revendication 1.

US2002082518 divulge un porte-outil selon le préambule de la revendication 12.

J L'un des buts de l'invention est donc de concevoir un système permettant l'orientation ou le déplacement rapide d'un porte-outil (porte-endoscope, porte-aiguille ou trocart), présentant un encombrement minimal.

Ce système doit également permettre de faire varier continûment les positions du porte-outil.

Un autre but de l'invention est qu'un tel système puisse être compatible avec les environnements de tomographie et de résonance magnétique.

Un autre but de l'invention est de procurer un système léger et garantissant la sécurité du patient en cas de génération d'efforts susceptibles de le blesser.

### BREVE DESCRIPTION DE L'INVENTION

Conformément à l'invention, il est proposé un dispositif pour positionner ou/et orienter dans le corps d'un patient, comprenant :
- une plateforme apte à être placée sur le corps du patient,
- un support orientable dont au moins une partie est rigidement liée au porte-outil,
- des moyens de guidage en rotation du support orientable par rapport à la plateforme dans deux directions d'un plan parallèle à la plateforme,
- au moins deux actionneurs pneumatiques coopérant avec ledit support orientable de sorte qu'une variation de pression de l'un au moins des actionneurs pneumatiques engendre une rotation du support orientable dans la première direction et/ou la deuxième direction,
dans lequel lesdits actionneurs pneumatiques sont des volumes déformables dans une direction déterminée en fonction de la pression interne, présentant chacun une face solidaire de la plateforme ou des moyens de guidage et une face solidaire du support orientable, de sorte qu'une variation de la pression interne entraîne un déplacement de la face solidaire du support orientable.

Selon un premier mode de réalisation de l'invention, les actionneurs pneumatiques sont au nombre de quatre, le support orientable comprend deux paires de plaques, les deux plaques de chaque paire étant parallèles entre elles et orthogonales aux plaques de l'autre paire, et les actionneurs pneumatiques sont agencés par paires antagonistes de telle sorte que la pression interne d'un actionneur solidaire d'une plaque du support orientable varie en sens inverse de la pression interne de l'actionneur solidaire de la plaque opposée à ladite plaque.

De manière particulièrement avantageuse, les moyens de guidage comprennent deux guides semi-circulaires disposés longitudinalement dans la première direction et une première paire de plaques du support orientable présente des moyens pour coulisser le long desdits guides.

Lesdits guides semi-circulaires sont en outre mobiles en rotation autour de la première direction, et solidaires de la deuxième paire de plaques du support orientable.

Selon un deuxième mode de réalisation de l'invention, les actionneurs pneumatiques sont au nombre de trois et le support orientable est un prisme à base triangulaire, de sorte que chacun des actionneurs est solidaire de l'une des trois faces latérales du support orientable.

Par ailleurs, le porte-outil comprend avantageusement des moyens de guidage en translation de l'outil dans une direction parallèle à l'axe du porte-outil, lesdits moyens de guidage de l'outil coopérant avec des actionneurs pneumatiques déformables dans une direction déterminée en fonction de la pression interne, de sorte qu'une variation de pression de l'un au moins desdits actionneurs pneumatiques engendre une translation de l'outil.

Si l'axe de translation de l'outil est confondu avec l'axe du porte-outil, lesdits actionneurs pneumatiques sont de préférence des cylindres toroïdaux, de sorte que l'outil coulisse à l'intérieur des tores.

Si l'axe de translation de l'outil est distinct de l'axe du porte-outil, lesdits actionneurs pneumatiques sont des cylindres.

Le dispositif comprend en outre des moyens pour relier la plateforme au bras d'un robot d'assistance chirurgicale ; lesdits moyens comprennent au moins une articulation rotoïde passive.

De manière particulièrement avantageuse, la plateforme, le support orientable, les actionneurs pneumatiques et les moyens de guidage en rotation du support orientable sont dans des matériaux non métalliques et non magnétiques, de sorte que ledit dispositif est apte à être utilisé en tomographie et/ou en résonance magnétique.

Selon un autre aspect, l'invention porte sur un porte-outil adapté pour être utilisé dans le dispositif qui vient d'être décrit.

Ledit porte-outil comprend des moyens d'entraînement en translation de l'outil selon un axe, un piston rigidement lié à l'outil, mobile en coulissement dans un boîtier selon une direction longitudinale parallèle audit axe. Lesdits moyens comprennent avantageusement deux actionneurs pneumatiques situés dans le boîtier de part et d'autre du piston, déformables dans une direction déterminée en fonction de la pression interne, et coopérant avec le piston de telle sorte qu'une variation de pression dans l'un au moins des actionneurs génère un coulissement du piston selon ladite direction.

Dans un mode particulier de réalisation du porte-outil, dans lequel l'axe de translation de l'outil et la direction de coulissement du piston sont confondus, les actionneurs pneumatiques sont des cylindres toroïdaux dont le diamètre intérieur est supérieur au diamètre de l'outil.

Selon une variante, l'axe de translation de l'outil et la direction de coulissement du piston sont distincts et le boîtier présente une fente longitudinale pour le coulissement d'un élément reliant le piston et l'outil.

### BREVE DESCRIPTION DES DESSINS

D'autres caractéristiques et avantages de l'invention ressortiront de la description détaillée qui va suivre, en référence aux dessins annexés sur lesquels :
- la figure 1 est une vue éclatée d'un premier mode de réalisation du dispositif conforme à l'invention,
- la figure 2 illustre un mode de fixation d'un actionneur sur la plate-forme,
- la figure 3 représente deux orientations possibles de la plate-forme en fonction de la respiration du patient,
- la figure 4 illustre le support orientable pour le porte-outil,
- la figure 5 présente les mouvements angulaires que peut adopter le porte-outil,
- la figure 6 illustre les positions extrêmes que peut atteindre le porte-outil selon l'un des degrés de liberté,
- la figure 7 représente le déplacement selon un premier degré de liberté du porte-outil sous l'effet du travail en antagonisme de deux actionneurs,
- la figure 8 illustre le déplacement selon un deuxième degré de liberté du porte-outil résultant du travail en antagonisme de deux autres actionneurs,
- la figure 9 illustre un premier mode de réalisation du porte-outil,
- la figure 10 illustre un deuxième mode de réalisation du porte-outil,
- la figure 11 illustre une variante de réalisation du dispositif conforme à l'invention,
- la figure 12 présente les forces appliquées au support orientable dans le dispositif de la figure 11,
- la figure 13 illustre de manière plus détaillée la construction du dispositif de la figure 11.

### DESCRIPTION DETAILLEE DE L'INVENTION

Par outil, on entend dans ce texte tout dispositif utilisable lors d'une intervention chirurgicale : il peut ainsi s'agir d'un instrument chirurgical (tel qu'une aiguille par exemple) ou bien un système d'imagerie médicale, tel qu'un endoscope ou un laparoscope

Par porte-outil, on entend dans ce texte un dispositif portant l'instrument chirurgical qui sera inséré dans le corps d'un patient. Selon l'instrument employé, le porte-outil pourra ainsi être qualifié de porte-aiguille, de porte-endoscope ou encore de trocart.

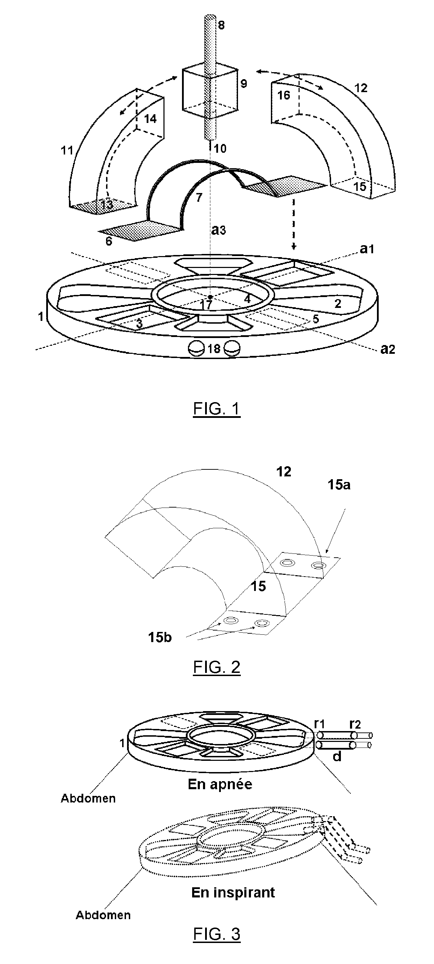

Lors d'une intervention chirurgicale sur un patient, telle qu'une ponction ou une laparoscopie, il est nécessaire d'orienter et de déplacer un outil (tel qu'une aiguille 10 ou un endoscope par exemple) porté par un porte-outil 8, comme illustré sur la figure 1.

Le dispositif conforme à l'invention permet au chirurgien d'orienter, par le moyen de deux rotations, le porte-outil 8 suivant les axes a1 et a2 et de translater l'outil suivant l'axe a3. L'orientation du porte-outil 8 est contrôlée par l'intermédiaire de l'orientation d'un support orientable 9, auquel le porte-outil 8 est rigidement lié.

Le porte-outil 8 est lié au support orientable 9 par des moyens mécaniques conventionnels tels que des vis ou des moyens de clipsage (c'est-à-dire par pression mécanique).

La figure d'ensemble 1 et les figures de détail 4, 5, 7 et 8 représentent un mode préféré, mais non limitatif, de réalisation de l'invention.

Selon ce mode préféré de réalisation, le dispositif comprend une plateforme circulaire 1 comprenant quatre espaces creux 2 afin de réduire le poids du système, deux espaces creux 3 pour recevoir les deux plateformes rectangulaires 6 et une cavité circulaire 4 définissant la zone de travail, c'est-à-dire la zone d'introduction de l'outil dans le corps du patient.

La plateforme 1 présente typiquement un diamètre de l'ordre de 10 cm.

Elle est posée sur le corps du patient, de sorte que la cavité circulaire 4 soit sensiblement centrée sur la zone d'introduction de l'outil. Dans le cas d'une ponction, le point 17 représente le point d'insertion de l'aiguille 10, invariante sous les rotations mentionnées précédemment.

La plateforme 1 est sensiblement plane, et les axes a1 et a2 selon lesquels le porte-outil 8 est orientable sont situés dans un plan parallèle au plan de la plateforme.

La plateforme 1 peut être tenue par un bras mécanique externe, rigide ou pas (non représenté), d'un robot d'assistance chirurgicale, pouvant s'adapter convenablement aux applications médicales envisagées. La liaison entre la plateforme et ce bras sera décrite plus bas.

Le dispositif comprend par ailleurs deux plateformes 6 reliées par deux guides semi-circulaires 7 permettant de guider le déplacement angulaire du porte-outil 8 par une rotation autour de l'axe a2 grâce au support orientable 9.

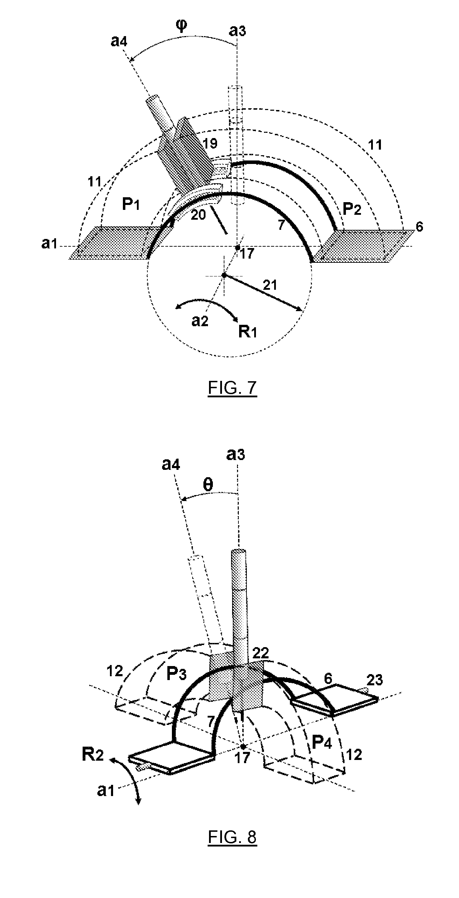

Comme on le verra dans la description de la figure 8, l'ensemble constitué par les plateformes 6 et les guides semi-circulaires n'est pas rigidement lié à la plateforme 1 mais mobile en rotation autour de l'axe a1 au moyen de boulons lisses.

Les mouvements d'orientation du porte-outil 8 sont assurés par des actionneurs pneumatiques 11 et 12 qui sont des volumes déformables dans une direction privilégiée en fonction de la pression interne.

On entend par là que c'est l'enveloppe définissant ledit volume qui se déforme, au contraire d'un actionneur pneumatique classique dans lequel l'enveloppe cylindrique est rigide et dont la forme ne varie pas.

Par ailleurs, le mouvement produit par un actionneur pneumatique classique est toujours linéaire tandis que celui produit par les actionneurs pneumatiques à volume déformable suit une direction préférentielle définie par la forme de l'actionneur.

L'enveloppe des actionneurs est par exemple fabriquée en plastique souple.

Ici, les actionneurs se présentent sous la forme de portions de tores, dont la fibre neutre décrit environ un demi-cercle. Les actionneurs sont aptes à s'allonger ou à se rétracter selon la fibre neutre.

Deux actionneurs 11 (dont un seul est représenté sur la figure 1) sont assemblés sur les deux plateformes rectangulaires 6 grâce aux surfaces 13 et sur le support orientable 9 grâce aux surfaces 14. Une variation de pression de l'un des actionneurs 11 permet donc d'incliner le support orientable 9 dans un plan contenant l'axe a1.

De même, deux actionneurs 12 (dont un seul est représenté sur la figure 1) sont assemblés sur les deux zones rectangulaires 5 de la plateforme 1 grâce aux surfaces 15 et sur le support orientable 9 grâce aux surfaces 16, permettant ainsi d'incliner le support orientable 9 selon un plan contenant l'axe a2.

En référence à la figure 2, l'assemblage de chaque actionneur peut être réalisé au moyen de vis en plastique logées dans des orifices 15b permettant de solidariser des languettes 15a situées à l'extérieur de l'actionneur 12 dans le prolongement de la surface 15, ou bien par un moyen de clipsage mécanique.

Chacun des actionneurs est relié à un circuit d'air comprimé (non représenté) muni de moyens pour contrôler la pression dans chacun d'eux. La gestion des pressions dans les différents actionneurs en fonction de l'orientation souhaitée du porte-outil est assurée par une interface électronique (non représentée).

Chaque paire d'actionneurs du même type travaille en antagonisme, c'est-à-dire que lorsque la pression d'air comprimé augmente dans un des actionneurs, celle de son actionneur antagoniste diminue et vice-versa. Ceci permet d'équilibrer les mouvements du support orientable 9. Toutefois, on comprend qu'un seul actionneur 11 et un seul actionneur 12 suffiraient pour incliner le support orientable 9.

On comprend donc que ces nouveaux actionneurs permettent, contrairement aux actionneurs pneumatiques classiques de type piston-cylindre, d'éviter le phénomène de « stick-slip », garantissant ainsi un fonctionnement fluide du système.

Les deux trous 18 montrés dans la plateforme 1 de la figure 1 servent à introduire deux cylindres ou doigts d associés à deux articulations rotoïdes passives r1 et r2 permettant le mouvement libre du système produit, par exemple, par la respiration du patient comme illustré à la figure 3.

Les deux doigts d sont reliés à un bras robotisé permettant une translation du robot dans l'espace tridimensionnel.

En effet, le dispositif est interdépendant du corps du patient et modifie l'orientation de la plateforme 1 en fonction des mouvements naturels du patient.

La figure 3 illustre deux instants où l'orientation de la plateforme 1 doit évoluer librement et naturellement, respectivement en apnée et pendant l'inspiration.

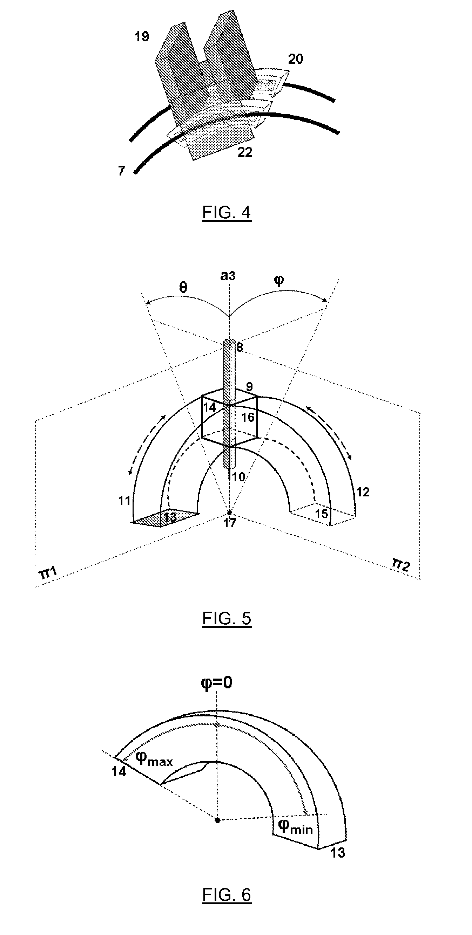

La figure 4 illustre de manière plus détaillée le support orientable 9 qui comprend deux plaques 19 servant à recevoir les deux surfaces de contact 14 des deux actionneurs 11.

Ces plaques 19 sont liées rigidement aux deux suiveurs 20 portant des billes sphériques afin de réduire le frottement entre les suiveurs 20 et les surfaces internes des guides 7.

Les deux plaques 22 servent à recevoir les deux surfaces de contact 16 de deux actionneurs 12 et sont reliées rigidement aux surfaces externes des guides semi-circulaires 7. Les plaques 22 sont centrées par rapport aux guides 7, c'est-à-dire qu'elles sont symétriques par rapport à un plan perpendiculaire au plan de la plateforme et comprenant l'axe a2.

On remarquera que les plaques 19 et 22 n'étant pas rigidement liées, le porte-outil 8 n'est rigidement lié qu'à une ou deux des plaques 19, mais pas aux plaques 22.

L'orientation du porte-outil 8 est définie par deux mouvements angulaires dans deux plans perpendiculaires π1 et π2, comme illustré à la figure 5. Le plan π1 est le plan perpendiculaire au plan de la plateforme et comprenant l'axe a1 ; le plan π2 est le plan perpendiculaire au plan de la plateforme et comprenant l'axe a2.

L'axe a3 montré sur la figure 5 est la position initiale (perpendiculaire à la plateforme) à partir de laquelle le porte-outil 8 doit être orienté.

Les deux mouvements angulaires sont donc associés respectivement aux anges ϕ et θ mesurés à partir de l'axe a3 montré à la figure 5.

A partir de sa position initiale ϕ=0 et θ=0, le porte-outil 8 peut, par l'intermédiaire des actionneurs antagonistes, atteindre pour chacun des degrés de liberté ϕ ou θ, une position maximale ϕₘₐₓ ou θₘₐₓ et une position minimale ϕₘᵢₙ ou θₘᵢₙ, comme il est illustré dans la figure 6 pour l'angle ϕ et pour l'un des actionneurs 11 avec les surfaces de contact 13 et 14.

La figure 7 illustre le mouvement produit sur le porte-outil par le travail en antagonisme des deux actionneurs 11 dessinés en pointillés.

Dans le cas présenté, la pression d'air comprimé P1 de l'actionneur de gauche est inférieure à la pression P2 de l'actionneur de droite.

Cette différence de pression permet aux suiveurs 20 de se déplacer sur les guides 7, changeant ainsi la position angulaire du porte-outil.

L'angle généré ϕ est donc mesuré entre l'axe a3 associé à la position initiale et l'axe a4 associé à la position finale du porte-outil.

Ce mouvement angulaire est défini par une rotation R1 effectuée par rapport à l'axe a2 de la figure 7.

Il est possible d'observer dans cette figure que la circonférence imaginaire de rayon 21 sert à concevoir le guide 7 et que l'axe a2 se trouve plus bas que le plan des plateformes rectangulaires 6. En effet, l'arc de circonférence définissant les guides 7 est conçu et placé de telle sorte que le point d'insertion 17 de l'aiguille 10 se trouve sur le plan inférieur de la plateforme 1 coïncidant avec la peau du patient.

Le même principe d'actionnement est utilisé pour produire la rotation d'angle θ du porte-outil, par le travail en antagonisme des deux actionneurs 12 dessinés en pointillés sur la figure 8.

Lorsque les pressions P3 et P4 sont égales, la position du porte-outil est celle montrée sur la figure 8 par des traits en continu.

Si P4 augmente de la même valeur que P3 diminue, le porte-outil se déplace d'un angle θ et atteint sa position finale dessinée en pointillés dans cette figure.

Le porte-outil tourne par rapport à l'axe a1 définissant une rotation R2.

L'ensemble rigide des deux plateformes rectangulaires 6 et des deux guides circulaires 7 tourne avec le porte-outil grâce aux deux plaques 22 recevant les deux surfaces de contact 16 des actionneurs 12 et aux deux boulons lisses 23 assurant leur assemblage avec la plateforme 1.

L'angle généré θ est mesuré entre l'axe a3 associé à la position initiale et l'axe a4 associé à la position finale du porte-outil.

De par sa conception, le dispositif permet de faire varier continûment la position angulaire du porte-outil, avec une grande rapidité, de telle sorte que la réponse du système ne présente pratiquement pas de retard.

La conception du porte-outil relève du même principe que celle du dispositif de positionnement, à savoir un actionnement pneumatique par des volumes déformables dans une direction déterminée en fonction de leur pression interne.

Le porte-outil se présente alors comme un boîtier, par exemple cylindrique, à l'intérieur duquel est agencé un piston rigidement lié à l'outil et entouré par deux actionneurs pneumatiques déformables, de telle sorte qu'une variation de pression de l'un au moins des actionneurs entraîne le coulissement du piston dans le boîtier. Selon les besoins, l'axe de translation de l'outil peut être confondu avec celui de coulissement du piston, ou bien distinct de celui-ci.

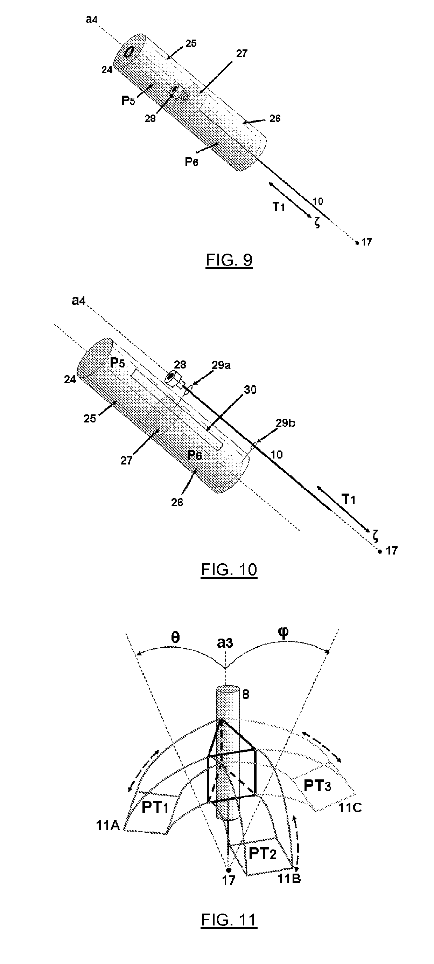

La figure 9 illustre une des conceptions possibles du porte-outil 8.

Ce porte-outil contient l'outil chirurgical tel qu'une aiguille 10 ou un endoscope ainsi que les éléments garantissant la translation T1 de cet outil chirurgical, suivant la direction ζ au long de l'axe a4.

A l'intérieur du boîtier cylindrique 24 se trouvent deux actionneurs déformables 25 et 26 fabriqués en plastique souple et travaillant en antagonisme. Ces actionneurs sont des cylindres toroïdaux permettant le passage de l'outil placé sur le piston 27 servant d'interface entre les deux actionneurs.

Si l'outil est une aiguille, sa partie supérieure 28 peut être reliée à un autre dispositif par, par exemple, un cathéter.

La différence de pression entre les valeurs de P5 et P6 entraînera le déplacement du piston 27 et donc de l'outil chirurgical. La course de l'outil est définie dans ce cas par la longueur des actionneurs.

Une deuxième conception possible du porte-outil 8 est montrée à la figure 10 où l'axe de l'outil ne coïncide pas avec celui du porte-outil mais où ces deux axes sont parallèles.

Dans ce cas, les actionneurs 25 et 26 sont des cylindres simples travaillant avec le même principe que dans le cas précédent.

Le piston et le boîtier portent des guides circulaires 29a et 29b avec des diamètres en général différents servant à maintenir le parallélisme entre les axes de l'outil et celui du porte-outil.

Le guide 29a assemblé rigidement au piston 27 et à l'outil 10 peut se déplacer en translation à l'intérieur de la fente 30 pratiquée dans le boîtier 24. La course de l'outil est définie dans ce cas par la longueur de la fente 30.

Le guide 29b est fixe par rapport au porte-outil et guide le déplacement de l'outil.

Il va de soi que le porte-outil qui vient d'être décrit est un objet à part entière de la présente invention.

De manière particulièrement avantageuse, l'ensemble des composants du dispositif (notamment la plateforme, le support orientable, les actionneurs, le porte-outil) peut être dans des matériaux - par exemple du plastique - non métalliques et non magnétiques. Ainsi, le dispositif est compatible avec des environnements de résonance magnétique et de tomographie.

Par ailleurs, le dispositif ainsi constitué possède un encombrement particulièrement réduit, puisqu'il est compris dans un cube d'environ 10 cm de côté, ce qui simplifie son installation dans l'environnement opératoire.

Il présente également l'avantage d'être très léger. Ainsi, la masse du prototype mis au point par les inventeurs est inférieure à 1 kg.

Enfin, la compliance naturelle du dispositif joue en faveur de la sécurité du patient lors de la génération d'efforts susceptibles de blesser le patient. En effet, si l'outil chirurgical est une aiguille, les actionneurs pneumatiques déformables possèdent une souplesse suffisante pour permettre à l'aiguille, lorsqu'elle est à l'intérieur du corps du patient, de bouger suivant les mouvements de la respiration et des organes, ce qui permet de diminuer le risque de blessures. En revanche, si le système n'était pas compliant (ce serait le cas d'un système tenant l'aiguille rigidement, tel que les actionneurs pneumatiques de type piston-cylindre de l'art antérieur), les mouvements dus à la respiration et aux organes pourraient causer des blessures en rayant ou en perforant les organes. Ainsi, pour un système compliant, lorsqu'il existe un effort important agissant sur le système, celui-ci réagit suivant la direction de l'effort et ne s'oppose pas à cet effort.

Des configurations différentes pour l'actionnement avec des chambres pneumatiques peuvent être envisagées ; en particulier, concernant la géométrie des actionneurs pneumatiques et leur disposition.

La figure 11 illustre ainsi une variante de réalisation de l'invention comprenant une disposition triangulaire des actionneurs 11 A, 11B et 11C qui permet, en fonction des trois valeurs de pression PT1, PT2 et PT3, l'obtention d'une position finale du porte-outil 8 associée à une combinaison des valeurs des angles ϕ et θ.

Dans ce dispositif à trois actionneurs, ceux-ci sont placés à 120° les uns des autres.

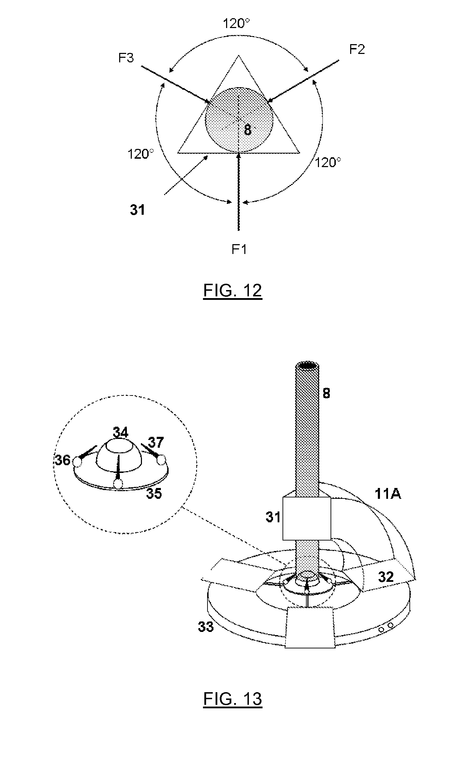

Le porte-outil reçoit donc, par l'action de trois pressions sur trois surfaces de contact, trois forces (F1, F2 et F3) agissant vers l'axe du porte-outil, comme illustré à la figure 12.

Lorsque ces forces ont la même valeur (c'est-à-dire que la force résultante est nulle), l'axe du porte-outil est à la verticale.

Une articulation rotoïde placée à proximité du point d'insertion de l'outil permet au porte-outil de s'orienter suivant la direction de la force résultante.

Le principe de fonctionnement reste similaire au cas de quatre actionneurs où des plaques de contact sont utilisées pour assurer la transmission de puissance grâce aux pressions pneumatiques dans chaque actionneur.

Le porte-outil 8 est alors une sorte de mât rigidement lié à un support orientable se présentant sous la forme d'une structure prismatique triangulaire 31 pour recevoir trois surfaces de contact, une pour chacun des trois actionneurs.

Sur la figure 13, on peut ainsi observer l'actionneur 11A en contact d'une part avec le prisme triangulaire 31 et d'autre part avec l'une des trois plaques 32 reposant sur la plateforme 33 similaire à la plateforme 1 de la figure 1.

Le porte-outil reste en contact avec la surface sphérique 34 (qui constitue une grande articulation rotoïde) grâce aux trois éléments mécaniques 37 télescopiques qui se plient ou s'allongent en fonction des combinaisons des pressions dans les trois chambres pneumatiques.

Ces éléments télescopiques 37 sont attachés au porte-outil 8 et à la plateforme 35 par des articulations rotoïdes passives 36. Les plateformes 33 et 35 sont liées par trois éléments rigides montrés dans la même figure.

Sur le bord de la plateforme 33, il est également possible d'observer les deux trous servant à recevoir les doigts d présentés à la figure 3.

Enfin, il va de soi que les exemples que l'on vient de donner ne sont que des illustrations particulières en aucun cas limitatives quant aux domaines d'application de l'invention.

## Revendications

1. Dispositif pour positionner ou/et orienter dans le corps d'un patient un outil chirurgical (10) porté par un porte-outil (8), ledit dispositif comprenant :
- une plateforme (1) apte à être placée sur le corps du patient,
- un support orientable (9) dont au moins une partie est adaptée pour être rigidement liée au porte-outil (8),
- des moyens (6, 7, 20, 23) de guidage en rotation du support orientable (9) par rapport à la plateforme (1) autour de deux axes (a1, a2) d'un plan parallèle à la plateforme (1),
- au moins 2 actionneurs pneumatiques (11, 12; 11A, 11B, 11C) coopérant avec ledit support orientable (9) de sorte qu'une variation de pression de l'un au moins des actionneurs pneumatiques engendre une rotation du support orientable (9) dans la première direction (a1) et/ou la deuxième direction (a2),
**caractérisé en ce que** lesdits actionneurs pneumatiques (11, 12) sont des volumes déformables dans une direction déterminée en fonction de la pression interne, présentant chacun une face (13, 15) solidaire de la plateforme (1) ou des moyens de guidage (6), et une face (14, 16) solidaire du support orientable (9), de sorte qu'une variation de la pression interne entraîne un déplacement de la face (14, 16) solidaire du support orientable (9).

2. Dispositif selon la revendication 1, **caractérisé en ce que** :
- les actionneurs pneumatiques (11, 12) sont au nombre de quatre,
- le support orientable (9) comprend deux paires de plaques (19, 22), les deux plaques de chaque paire étant parallèles entre elles et orthogonales aux plaques de l'autre paire,
- les actionneurs pneumatiques sont agencés par paires antagonistes de telle sorte que la pression interne d'un actionneur solidaire d'une plaque (19, 22) du support orientable (9) varie en sens inverse de la pression interne de l'actionneur solidaire de la plaque opposée à ladite plaque.

3. Dispositif selon la revendication 2, **caractérisé en ce que** les moyens de guidage comprennent deux guides (7) semi-circulaires disposés longitudinalement dans la première direction (a1) et **en ce qu'**une première paire de plaques (19) du support orientable (9) présente des moyens (20) pour coulisser le long desdits guides (7).

4. Dispositif selon la revendication 3, **caractérisé en ce que** lesdits guides semi-circulaires (7) sont en outre mobiles en rotation autour de la première direction (a1).

5. Dispositif selon la revendication 4, **caractérisé en ce que** lesdits guides (7) sont solidaires de la deuxième paire de plaques (22) du support orientable (9).

6. Dispositif selon la revendication 1, **caractérisé en ce que** les actionneurs pneumatiques (11A, 11B, 11C) sont au nombre de trois et **en ce que** le support orientable (9) est un prisme à base triangulaire, de sorte que chacun des actionneurs est solidaire de l'une des trois faces latérales du support orientable (9).

7. Dispositif selon l'une des revendications 1 à 6, **caractérisé en ce qu'**il comporte un porte-outil (8) comprenant des moyens de guidage en translation de l'outil (10) dans une direction parallèle à l'axe (a4) du porte-outil (8), lesdits moyens de guidage de l'outil (10) coopérant avec des actionneurs pneumatiques (25, 26) déformables dans une direction déterminée en fonction de la pression interne, de sorte qu'une variation de pression de l'un au moins desdits actionneurs pneumatiques engendre une translation de l'outil (10).

8. Dispositif selon la revendication 7, **caractérisé en ce que** l'axe de translation de l'outil (10) est confondu avec l'axe (a4) du porte-outil (8) et **en ce que** lesdits actionneurs pneumatiques (25, 26) sont des cylindres toroïdaux, de sorte que l'outil (10) coulisse à l'intérieur des tores.

9. Dispositif selon la revendication 8, **caractérisé en ce que** l'axe de translation de l'outil (10) est distinct de l'axe (a4) du porte-outil (8) et **en ce que** lesdits actionneurs pneumatiques (25, 26) sont des cylindres.

10. Dispositif selon l'une des revendications 1 à 9, **caractérisé en ce qu'**il comprend des moyens (d) pour relier la plateforme (1) au bras d'un robot d'assistance chirurgicale et **en ce que** lesdits moyens (d) comprennent au moins une articulation rotoïde passive (r1, r2).

11. Dispositif selon l'une des revendications 1 à 10, **caractérisé en ce que** la plateforme (1), le support orientable (9), les actionneurs pneumatiques (11, 12 ; 11A, 11 B, 11 C) et les moyens (6, 7, 20, 23) de guidage en rotation du support orientable (9) sont dans des matériaux non métalliques et non magnétiques, de sorte que ledit dispositif est apte à être utilisé en tomographie et/ou en résonance magnétique.

12. Porte-outil (8) adapté pour être utilisé dans le dispositif selon l'une des revendications précédentes, comprenant des moyens d'entraînement en translation de l'outil (10) selon un axe (a4), ainsi qu'un piston (27) rigidement lié à l'outil (10), mobile en coulissement dans un boîtier (24) selon une direction longitudinale parallèle à l'axe (a4), **caractérisé en ce que** lesdits moyens comprennent deux actionneurs pneumatiques (25, 26) situés dans le boîtier (24) de part et d'autre du piston (27), déformables dans une direction déterminée en fonction de la pression interne, et coopérant avec le piston (27) de telle sorte qu'une variation de pression dans l'un au moins des actionneurs génère un coulissement du piston (27) selon ladite direction.

13. Porte-outil (8) selon la revendication 12, **caractérisé en ce que** l'axe (a4) de translation de l'outil (10) et la direction de coulissement du piston (27) sont confondus et **en ce que** les actionneurs pneumatiques (25, 26) sont des cylindres toroïdaux dont le diamètre intérieur est supérieur au diamètre de l'outil.

14. Porte-outil (8) selon la revendication 12, **caractérisé en ce que** l'axe (a4) de translation de l'outil (10) et la direction de coulissement du piston (27) sont distincts et **en ce que** le boîtier (24) présente une fente (30) longitudinale pour le coulissement d'un élément reliant le piston (27) et l'outil (10).

## Patentansprüche

1. Vorrichtung zum Positionieren oder/und Ausrichten im Körper eines Patienten eines von einem Instrumentenhalter (8) getragenen chirurgischen Instruments (10), wobei die Vorrichtung umfasst:
- eine Plattform (1), die auf dem Körper des Patienten platzierbar ist,
- eine ausrichtbare Stütze (9), von der mindestens ein Teil ausgebildet ist, um mit dem Instrumentenhalter (8) starr verbunden zu sein,
- Rotationsführungsmittel (6, 7, 20, 23) der im Verhältnis zur Plattform (1) um zwei Achsen (a1, a2) einer zur Plattform (1) parallelen Ebene ausrichtbaren Stütze (9),
- mindestens 2 pneumatische Aktuatoren (11, 12; 11A, 11B, 11C), die mit der ausrichtbaren Stütze (9) derart zusammenarbeiten, dass eine Druckschwankung mindestens einer der pneumatischen Aktuatoren eine Rotation der ausrichtbaren Stütze (9) in die erste Richtung (a1) und/oder die zweite Richtung (a2) erzeugt,
**dadurch gekennzeichnet, dass** die pneumatischen Aktuatoren (11, 12) in eine bestimmte Richtung in Abhängigkeit vom Innendruck verformbare Volumen sind, die jeweils eine mit der Plattform (1) oder den Führungsmitteln (6) fest verbundene Fläche (13, 15) aufweisen, und eine mit der ausrichtbaren Stütze (9) derart fest verbundene Fläche (14, 16), dass eine Schwankung des Innendrucks zu einer Translation der mit der ausrichtbaren Stütze (9) fest verbundenen Fläche (14, 16) führt.

2. Vorrichtung nach Anspruch 1, **dadurch gekennzeichnet, dass**:
- die pneumatische Aktuatoren (11, 12) anzahlmäßig vier sind,
- die ausrichtbare Stütze (9) zwei paarige Platten (19, 22) umfasst, wobei die zwei Platten jedes Paars parallel zueinander und orthogonal zu den Platten des anderen Paars sind,
- die pneumatischen Aktuatoren je nach Paaren derart antagonistisch ausgebildet sind, dass der Innendruck eines mit einer Platte (19, 22) der ausrichtbaren Stütze (9) fest verbundenen Aktuators in umgekehrter Richtung des Innendrucks des Aktuator schwankt, der mit der der Platte gegenüberliegenden Platte fest verbunden ist.

3. Vorrichtung nach Anspruch 2, **dadurch gekennzeichnet, dass** die Führungsmittel zwei halbkreisförmige Führungen (7) umfassen, die längs in der ersten Richtung (a1) angeordnet sind und dass ein erstes Paar Platten (19) der ausrichtbaren Stütze (9) Mittel (20) aufweist, um entlang der Führungen (7) zu gleiten.

4. Vorrichtung nach Anspruch 3, **dadurch gekennzeichnet, dass** die halbkreisförmigen Führungen (7) ferner rotierend um die erste Richtung (a1) bewegbar sind.

5. Vorrichtung nach Anspruch 4, **dadurch gekennzeichnet, dass** die Führungen (7) mit dem zweiten Paar Platten (22) der ausrichtbaren Stütze (9) fest verbunden sind.

6. Vorrichtung nach Anspruch 1, **dadurch gekennzeichnet, dass** die pneumatischen Aktuatoren (11A, 11B, 11C) anzahlmäßig drei sind und dass die ausrichtbare Stütze (9) ein Prisma mit einer dreieckigen Basis ist, so dass jeder der Aktuatoren mit einer der drei Seitenflächen der ausrichtbaren Stütze (9) fest verbunden ist.

7. Vorrichtung nach einem der Ansprüche 1 bis 6, **dadurch gekennzeichnet, dass** sie einen der Instrumentenhalter (8) aufweist, der Translationsführungsmittel des Instruments (10) in einer zur Achse (a4) des Instrumentenhalters (8) parallelen Richtung umfasst, wobei die Führungsmittel des Instruments (10) mit in einer bestimmten Richtung in Abhängigkeit vom Innendruck verformbaren pneumatischen Aktuatoren (25, 26) zusammenarbeiten, so dass eine Schwankung des Drucks mindestens einer der pneumatischen Aktuatoren zu einer Translation des Instruments (10) führt.

8. Vorrichtung nach Anspruch 7, **dadurch gekennzeichnet, dass** die Translationsachse des Instruments (10) mit der Achse (a4) des Instrumentenhalters (8) zusammenfällt und dass die pneumatischen Aktuatoren (25, 26) torusförmige Zylinder sind, so dass das Instrument (10) in den Tori gleitet.

9. Vorrichtung nach Anspruch 8, **dadurch gekennzeichnet, dass** sich die Translationsachse des Instruments (10) von der Achse (a4) des Instrumentenhalters (8) unterscheidet und dass die pneumatischen Aktuatoren (25, 26) Zylinder sind.

10. Vorrichtung nach einem der Ansprüche 1 bis 9, **dadurch gekennzeichnet, dass** sie Mittel (d) umfasst, um die Plattform (1) mit dem Arm eines chirurgischen Assistenzroboters zu verbinden und dass die Mittel (d) mindestens ein passives rotoides Gelenk (r1, r2) umfassen.

11. Vorrichtung nach einem der Ansprüche 1 bis 10, **dadurch gekennzeichnet, dass** die Plattform (1), die ausrichtbare Stütze (9), die pneumatischen Aktuatoren (11, 12; 11A, 11B, 11C) und die Rotationsführungsmittel (6, 7, 20, 23) der ausrichtbaren Stütze (9) aus nichtmetallischen und nicht magnetischen Materialien sind, so dass die Vorrichtung imstande ist, bei einer Tomographie oder und/oder Magnetresonanztomografie verwendet zu werden.

12. Instrumentenhalter (8), der für die Benutzung in der Vorrichtung nach einem der vorangehenden Ansprüche ausgebildet ist, der Translationsantriebsmittel des Instruments (10) gemäß einer Achse (a4) umfasst, sowie einen starr mit dem Instrument (10) verbundenen Kolben (27), der sich in einem Gehäuse (24) gemäß einer Längsrichtung parallel zur Achse (a4) gleitend bewegt, **dadurch gekennzeichnet, dass** die Mittel zwei pneumatische Aktuatoren (25, 26) umfassen, die sich in dem Gehäuse (24) auf der einen und der anderen Seite des Kolbens (27) befinden, die in einer in Abhängigkeit vom Innendruck bestimmten Richtung verformbar sind und mit dem Kolben (27) derart zusammenarbeiten, dass eine Druckschwankung in mindestens einem der Aktuatoren ein Gleiten des Kolbens (27) gemäß der Richtung erzeugt.

13. Instrumentenhalter (8) nach Anspruch 12, **dadurch gekennzeichnet, dass** die Translationsachse (a4) des Instruments (10) und die Verschieberichtung des Kolbens (27) zusammenfallen und dass die pneumatischen Aktuatoren (25, 26) torusförmige Zylinder sind, deren Innendurchmesser größer als der Durchmesser des Instruments sind.

14. Instrumentenhalter (8) nach Anspruch 12, **dadurch gekennzeichnet, dass** die Translationsachse (a4) des Instruments (10) und die Verschieberichtung des Kolbens (27) unterschiedlich sind und dass das Gehäuse (24) einen länglichen Schlitz (30) für das Gleiten eines Elements aufweist, das den Kolben (27) und das Instrument (10) verbindet.

## Claims

1. A device for positioning or/and orienting in the body of a patient a surgical tool (10) borne by a tool holder (8), said device comprising:
- a platform (1) capable of being placed on the body of the patient,
- an orientable support (9), at least one portion of which is adapted to be rigidly bound to the tool holder (8),
- means (6, 7, 20, 23) for guiding in rotation the orientable support (9) with respect to the platform (1) about two axes (a1, a2) of a plane parallel to the platform (1),
- at least two pneumatic actuators (11, 12; 11A, 11B, 11C) cooperating with said orientable support (9) so that a change in pressure of at least one of the pneumatic actuators generates a rotation of the orientable support (9) in the first direction (a1) and/or the second direction (a2),
**characterized in that** said pneumatic actuators (11, 12) are volumes deformable in a determined direction depending on the internal pressure, each having a face (13, 15) attached to the platform (1) or guiding means (6) and a face (14, 16) attached to the orientable support (9), so that a change in the internal pressure causes displacement of the face (14, 16) attached to the orientable support (9).

2. The device according to claim 1, **characterized in that**:
- the pneumatic actuators (11, 12) are four in number,
- the orientable support (9) comprises two pairs of plates (19, 22), both plates of each pair being parallel to each other and orthogonal to the plates of the other pair,
- the pneumatic actuators are laid out in antagonistic pairs so that the internal pressure of an actuator firmly attached to a plate (19, 22) of the orientable support (9) varies in the opposite direction to the internal pressure of the actuator firmly attached to the plate opposite said plate.

3. The device according to claim 2, **characterized in that** the guiding means comprise two semi-circular guides (7) positioned longitudinally in the first direction (a1) and **in that** a first pair of plates (19) of the orientable support (9) has means (20) for sliding along said guides (7).

4. The device according to claim 3, **characterized in that** said semi-circular guides (7) are further rotationally mobile around the first direction (a1).

5. The device according to claim 4, **characterized in that** said guides (7) are attached to the second pair of plates (22) of the orientable support (9).

6. The device according to claim 1, **characterized in that** the pneumatic actuators (11A, 11B, 11C) are three in number and **in that** the orientable support (9) is a prism with a triangular base, so that each of the actuators is attached to one of the three side faces of the orientable support (9).

7. The device according to any of claims 1 to 6, **characterized in that** it includes a tool holder (8) comprising means for guiding the tool (10) in translation in a direction parallel to the axis (a4) of the tool holder (8), said means for guiding the tool (10) cooperating with deformable pneumatic actuators (25, 26) in a determined direction depending on the internal pressure, so that a change in pressure of at least one of said pneumatic actuators generates translation of the tool (10).

8. The device according to claim 7, **characterized in that** the translation axis of the tool (10) coincides with the axis (a4) of the tool holder (8) and **in that** said pneumatic actuators (25, 26) are toroidal cylinders, so that the tool (10) slides inside the toruses.

9. The device according to claim 8, **characterized in that** the translation axis of the tool (10) is distinct from the axis (a4) of the tool holder (8) and **in that** said pneumatic actuators (25, 26) are cylinders.

10. The device according to any of claims 1 to 9, **characterized in that** it comprises means (d) for connecting the platform (1) to the arm of a surgical aid robot and **in that** said means (d) comprise at least one passive rotoid joint (r1, r2).

11. The device according to any of claims 1 to 10, **characterized in that** the platform (1), the orientable support (9), the pneumatic actuators (11, 12; 11A, 11B, 11C) and the means (6, 7, 20, 23) for guiding the orientable support (9) in rotation are in non-metal and non-magnetic materials, so that said device is able to be used in tomography and/or in magnetic resonance.

12. A tool holder (8) adapted to be used in the device according to any of the preceding claims, comprising means for driving the tool (10) in translation along an axis (a4), and a piston (27) rigidly bound to the tool (10), slidably mobile in a casing (24) along a longitudinal direction parallel to the axis (a4), **characterized in that** said means comprise two pneumatic actuators (25, 26) located in the casing (24) on either side of the piston (27), deformable in a determined direction depending on the internal pressure, and cooperating with the piston (27) so that a change in pressure in at least one of the actuators generates sliding of the piston (27) along said direction.

13. The tool holder (8) according to claim 12, **characterized in that** the translation axis (a4) of the tool (10) and the sliding direction of the piston (27) coincide and **in that** the pneumatic actuators (25, 26) are toroidal cylinders, the inner diameter of which is larger than the diameter of the tool.

14. The tool holder (8) according to claim 12, **characterized in that** the translation axis (a4) of the tool (10) and the sliding direction of the piston (27) are distinct and **in that** the casing (24) has a longitudinal slot (30) for sliding a member connecting the piston (27) and the tool (10).
